(19) **Europäisches Patentamt European Patent Office Office européen des brevets**

(11) **EP 1 663 059 B1**

(12) **EUROPEAN PATENT SPECIFICATION**

(45) Date of publication and mention
of the grant of the patent:
**13.02.2019 Bulletin 2019/07**

(51) Int Cl.:
***A61C 17/22*** *(2006.01)*      ***A61C 17/34*** *(2006.01)*
***A61C 19/06*** *(2006.01)*      ***A61N 5/06*** *(2006.01)*

(21) Application number: **04788641.1**

(86) International application number:
**PCT/US2004/029335**

(22) Date of filing: **09.09.2004**

(87) International publication number:
**WO 2005/023143 (17.03.2005 Gazette 2005/11)**

(54) **KIT COMPRINSING AN ILLUMINATED ELECTRIC TOOTHBRUSH**

**KI MIT EINER BELEUCHTETEN ELEKTRISCHEN ZAHNBÜRSTE**

**KIT COMPENANT UNE BROSSE A DENTS ELECTRIQUE LUMINESCENTE**

(84) Designated Contracting States:
**AT BE BG CH CY CZ DE DK EE ES FI FR GB GR
HU IE IT LI LU MC NL PL PT RO SE SI SK TR**

(30) Priority: **09.09.2003   US 501266 P
26.04.2004   US 832168
10.05.2004   US 842302
17.05.2004   US 847429
09.07.2004   US 887667
09.07.2004   US 887644
09.07.2004   US 888206**

(43) Date of publication of application:
**07.06.2006   Bulletin 2006/23**

(73) Proprietor: **THE PROCTER & GAMBLE COMPANY
Cincinnati, OH 45202 (US)**

(72) Inventors:
• **GHOSH, Chanchal, Kumar
West Chester, Ohio 45069 (US)**
• **MAJETI, Satyanarayana
Middletown, Ohio 45044 (US)**
• **PINYAYEV, Aleksey, Mikhailovich
West Chester, Ohio 45069 (US)**
• **CHAN, John, Geoffrey
Maineville, Ohio 45039 (US)**
• **WILLEY, Alan, David
Cincinnati, Ohio 45215 (US)**

(74) Representative: **Töpert, Verena Clarita
Procter & Gamble Service GmbH
IP Department
Sulzbacher Straße 40-50
65824 Schwalbach am Taunus (DE)**

(56) References cited:
**US-A- 5 989 569          US-A1- 2003 059 738
US-A1- 2003 104 340**

**Description**

**Field of the Invention**

The present invention relates to illuminated electric toothbrushes that utilize a light emitting element. More particularly, the present invention relates to a kit comprising an electric toothbrush comprising an electrically powered element in combination with a substance which can be used to treat the surfaces of the oral cavity.

**Background of the Invention**

Plaque, gingivitis, periodontal disease, and discoloration of the teeth are all undesirable conditions that affect many people. In particular white teeth have long been considered cosmetically desirable. Unfortunately, due to the presence of chromogenic substances in the food, beverages, tobacco, and salivary fluid, in addition to internal sources such as blood, amalgam restoratives, and antibiotics such as tetracycline, teeth often become discolored. Many of the existing strategies available for removing or destroying teeth stains include hydrogen peroxide in order to attack the chromogen molecules in such a way as to render them colorless, water-soluble or both. Additionally, some whitening procedures include the use of light and/or lasers in combination with certain chemical substances. However, the use of light of sufficient intensity to deliver an oral care benefit such as whitening has previously required the consumer to seek a professional in-office procedure. Currently available outside the office treatments include the use of lower concentrations of hydrogen peroxide on trays and/or strips. However, the majority of the outside of the office treatments do not use a light, in particular a light of sufficient intensity to result in the desired oral care benefit. Therefore, a recognized consumer need is a low cost commercial oral care system utilizing light that can deliver light of a quality and quantity that can in turn deliver the desired oral care benefit when used by the consumer at home.

US 2003/0059738 A1 discloses a method and material for self-cleaning of the teeth and mouth using a source of light in the visible range in conjunction with a photosensitive oral hygiene composition possessing a broad absorption spectrum in the visible range. US 2003/0059738 A1 involves the use of a light-providing dental device to activate a photosensitive agent to and destroy harmful bacteria in the oral cavity. It may prevent or deter oral diseases, inflammations, and infections.

US 2003/104340 A1 discloses a light-emitting toothbrush comprising a head having a plurality of bristles affixed to and extending from a first side, a handle detachably affixed to a second side of the head, a light guide having a first end disposed within the head and a second end disposed within the handle, and a light source optically coupled to the second end of the light guide. The light source emits light having a spectrum ranging from about 350 nanometers to about 700 nanometers. Light from the light source entering the light guide is guided to the first end of the light guide and out of the first side of the head. The light emitted from the head illuminates teeth while brushing.

US 5,989,569 A discloses a delivery system for an oral care substance that includes a strip of material having a yield point and thickness such that the strip of material substantially conforms to a shape of a tooth via permanent deformation under a pressure less than about 250,000 Pascals when the delivery system is placed there against. The delivery system also includes an oral care substance applied to the strip of material such that when the delivery system is placed on a surface of the tooth, the substance contacts the surface providing an active onto the surface. The substance also provides adhesive attachment between the strip of material and the surface to hold the delivery system in place for a sufficient time to allow the active to act upon the surface. The substance has an extrusion resistance sufficient to withstand a normal force applied to deform the strip of material so that the substance is not substantially extruded from between the strip of material and the surface during manual deformation of the strip of material. A method of delivery includes pre-coating the strip of material, having the wearer apply substance to the strip of material, or having the wearer apply the substance directly to the surface before applying a strip of material.

**Summary of the Invention**

The present invention provides a kit according to claim 1.

**Brief Description of the Drawings**

The invention may take physical form in certain parts and arrangements of parts, embodiments of which will be described in detail in this specification and illustrated in the accompanying drawings which form a part hereof, and wherein:

> **FIG. 1** is a perspective view of an electric toothbrush in accordance with the present invention.
> **FIG. 2** is a top planar view of the electric toothbrush of **FIG. 1.**

FIG. 2a is a top planar view of the electric toothbrush of **FIG. 1.**

FIG. 3 is a cross-sectional side elevational view of the electric toothbrush of **FIG. 1.**

FIG. 4 Is a cross-sectional side view of a head and neck of an embodiment of an electric toothbrush made according to the present invention.

FIG. 5 is a partial front elevational view of a head and neck of another embodiment of the present invention.

FIG. 6 is a partial front elevational view of a head and neck of yet another embodiment of the present invention.

FIG. 7 is a partial front elevational view of a head and neck of an embodiment not covered by the present invention.

FIG. 8 is a partial front elevational view of a head and neck of yet another embodiment of the present invention.

FIG. 9 is a partial front elevational view of a head and neck of an embodiment not covered by the present invention.

FIG. 10 is a partial front elevational view of a head and neck of still another embodiment of the present invention.

FIG. 11 is a perspective view of another embodiment of the illuminated electric toothbrush of the present invention in which the toothbrush includes a head and neck that can be separated from the handle.

FIG. 14 is a schematic of an electrical configuration suitable for use with the present invention.

FIG. 15 is a graph of the spectral distribution for a variety of colors for light-emitting elements that are suitable for use with the present invention.

FIG. 16 is a graph of the spectral distribution for a light-emitting element that emits a white light that is suitable for use with the present invention.

FIG. 17 is a graph illustrating a light radiation pattern suitable for use with the present invention.

FIG. 18 is a perspective view of a substantially flat strip of material having rounded corners.

FIG. 19 is a perspective view of an embodiment of the present invention, disclosing the flat strip of **FIG. 18** coated with an oral care substance.

FIG. 20 is a cross-sectional view disclosing the flat strip having a substance disposed thereon.

FIG. 21 is a cross-sectional view showing adjacent teeth having the strip of material of **FIG. 20** conforming and attached to the teeth.

FIG. 22 is a cross-sectional view showing the strip of material of **FIG. 20** attached to a single tooth.

FIG. 23 is a perspective view of a tray delivery device not covered by the present invention.

FIG. 24 is a cross-sectional view showing the tray of **FIG. 23** attached to a single tooth.

## Detailed Description of the Embodiments

Generally, the present invention relates to a kit comprising an electric toothbrush comprising a handle and a replacable head, the head comprising a movable bristle holder, and a light-emitting electrically powered element disposed thereon and a light activated tooth whitening substance coated on a delivery device in the form of a strip. The oral care implement having one or more electrically powered elements disposed on the head includes light emitting diodes, light-emitting elements using incandescent elements, laser elements, halogen elements, neon elements, fluorescent elements, plasma elements, xenon elements and/or any combination thereof. The oral care substance contains an active at a level where upon directed use, promotes the benefit desired by the user without detriment to the oral surfaces it is applied to. Examples of oral conditions these actives can address include appearance and structural change to teeth, whitening, stain bleaching, stain removal, plaque removal, tartar removal.

As used herein the term electrically powered element includes any electrically powered element that can convert electrical energy at the place where the element is disposed. For example, a light emitting element can convert electrical energy into light at the location where the element is disposed; such as on the head of an electric toothbrush.

The kit of the present invention comprises an electric toothbrush having a head, on which a light emitting element is disposed. Such electric toothbrushes can be used in personal hygiene to clean one's teeth and gums using a motorized movement, while the electrically powered element is activated. The present invention relates to an electric toothbrush having a replaceable or removable head and/or neck. Light can improve the whitening of teeth by making the teeth more susceptible to bleaching and/or activating a chemical in the oral care substance, thereby increasing the oral care benefit gained from the oral care substance alone. Examples of desired oral care benefits include, but are not limited to, whitening, stain bleaching, stain removal, plaque removal, and tartar removal. The electric toothbrush made according to the present invention is sold in a kit comprising oral care substances. Different oral care substances can be included in the kits, including, but not limited to, tooth whitening, stain removing and/or plaque removing.

## A. Oral Care Implement

The oral care implement of the present invention has one or more electrically powered elements disposed on the head including, but not limited to, light emitting diodes, light-emitting elements using incandescent elements, laser elements, halogen elements, neon elements, fluorescent elements, plasma elements, xenon elements and/or any combination thereof. Such oral care implements can include, but are not limited to, electric toothbrushes, powered flossers, tooth polishers, gum massagers, etc. The invention refers to an electric toothbrush comprising a light emitting element.

As used herein, the term "light" is intended to encompass the spectrum of both visible and non-visible (e.g., ultraviolet and infra-red) light. In one embodiment of the toothbrush of the present invention the light emitted from the light emitting element can be from about 370, 390, 410, 430, 450, 470, 490, 510, 530, 550, 570, 590, 610, 630, 650, 670, 690, 710 nm and/or less than about 770, 750, 730, 710, 690, 670, 650, 630, 610, 500, 400 nm. In another embodiment the light emitted can have a wavelength of greater than about 420, 430, 440, 450, 460, 470, 480, and/or 490 nm and/or less than about 490, 480, 470, 460, 450, 440, 430 nm. In yet another embodiment the light emitted can have a wave length from about 420, 430, 440, 450, 460, 470 nm and/or less than about 470, 460, 450, 430 nm. It will be appreciated that the particular range of wavelengths selected can depend upon the desired color of the light. In one embodiment the light emitted can be a blue color.

The oral care implement can also emit light of a particular intensity. Intensity can be either luminous intensity measured in candelas (or lumens/steradian), or flux density measured in Watts/meter$^2$. In one embodiment the flux density of the inventive illuminated electric toothbrush is from about 20, 30, 35, 40, 45, 50, 55, 60, 70, 100, 200, 250 mW/cm$^2$ and/or less than about 300, 250, 200, 150, 100, 70, 60, 50, 40, 30 mW/cm$^2$ or any combination of these.

In one embodiment, the electric toothbrush includes an elongated body portion or handle, a head, and a neck extending between the head and the handle. One or more light-emitting elements can be provided on the head on one or more moving bristle holders having a plurality of bristles thereon. The bristles may be formed into one or more groups or tufts. In certain embodiments, a light-emitting element can be positioned at the center or at the axis of movement of an oscillating bristle holder. Additionally, the light emitting element can act as the pin which serves as the axis and/or center of rotation for the movable bristle holder. The light-emitting element can be stationary, or it can be secured to the movable bristle holder so that the element moves with the bristle holder. The bristle holder can, in certain embodiments, feature a region, such as an aperture, which promotes the passage of light there through. That region may be formed from a transparent or translucent material, or alternatively, the region can be an aperture or other open area substantially free of bristles thereby permitting the passage of light. This region can be provided at any portion of the head of the toothbrush, including the center of a movable bristle holder.

The head includes a longitudinal axis, one or more moving bristle holders or carriers and, optionally, one or more static or fixed bristle holders. The moving bristle holders may rotate, swivel, gyrate, oscillate, linearly reciprocate, or undergo any combination of motions. The type of motion provided by the electric toothbrushes of the present invention can be widely varied. The static bristle holders and the arrangement of the static bristles disposed thereon can also be widely varied. For example, the static bristles might partially or wholly circumscribe the moving bristle holders or may be disposed in a gap between the moving bristle holders. Examples of some bristle holder motions and bristle arrangements suitable for use with the present invention are described in US 20030126699; US 20030084525; US 20030084524; US 20030084526; and WO 03/063723; and WO 03/063722. The bristles can be made from conventional non-elastomeric materials, such as polyethylene, or can be made from elastomeric materials such as natural or synthetic rubbers, polyolefins, polyetheramides, polyesters, styrenic polymers, polyurethanes, etc., or a combination of materials.

The handle has a hollow portion with a motor disposed therein that is operably connected to the moving bristle holders. A shaft extends from the motor through the neck and into at least a portion of the head. The shaft may rotate, oscillate, linearly reciprocate, gyrate, orbit, or move in a conical fashion when driven by the motor in order to impart one or more motions to the moving bristle holders. A gearing arrangement can be provided between the motor and the shaft or between the shaft and the moving bristle holders in order to impart motion thereto. Exemplary shaft and/or gearing arrangements are shown in U.S. Patent Nos. 6,360,395 and 5,617,601, and U.S. Patent Application Nos. 2003/0134567 and 2003/0163881. The handle also has a power source, such as one or more batteries, disposed therein for powering the motor and the light-emitting elements. Alternatively, the electric toothbrush may be connected to an external power source for powering the motor. A switch is disposed on the handle for activating the motor and/or light-emitting elements. The switch includes an actuator button and a metal contact. The switch is manually depressed by pressing a molded actuator button down, which presses against a metal contact, completing the circuit, as in a conventional momentary switch. The switch allows continuous operation, through a ramp design, by depressing and sliding the actuator button forward as in a conventional continuous switch. By combining these two functions in one switch, the consumer can try the unit and see its operation prior to purchase, and still operate it continuously once out of the package. The switch can also activate one or more light emitting elements. The light emitting elements are energized whenever the motor is activated, however, the electric toothbrush can also have a switch designated to activate the light emitting element.

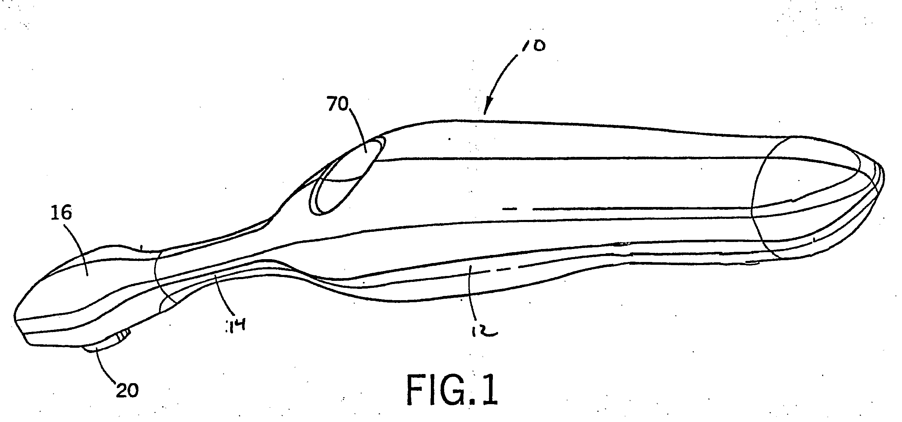

Referring now to the drawings wherein the showings are for the purposes of illustrating the embodiments of the invention only and not for purposes of limiting same, **FIG. 1** shows an illuminated electric toothbrush **10** according to one embodiment of the present invention. The electric toothbrush can be used for personal hygiene such as brushing one's teeth and gums. As shown in **FIG. 1,** the electric toothbrush includes a handle **12** and a neck **14** attached to the handle **12.** A head **16** is attached to neck **14.** Typically, the head is larger than the neck **14,** which is also typically smaller than the handle **12.**

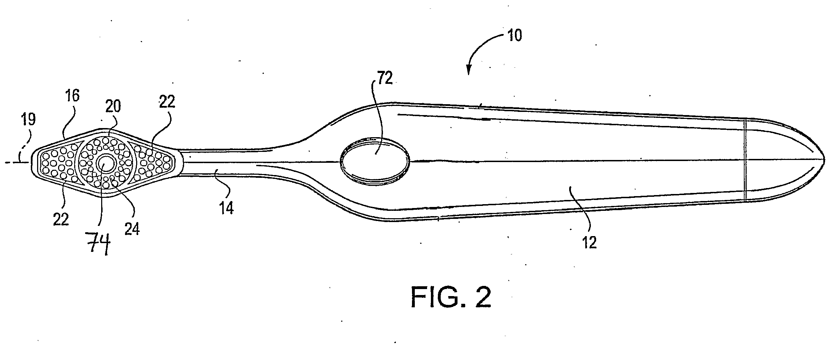

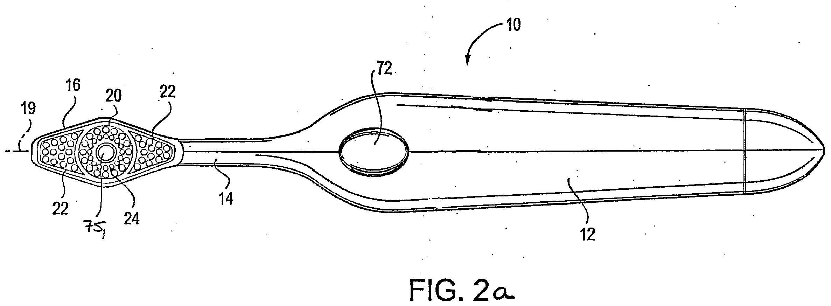

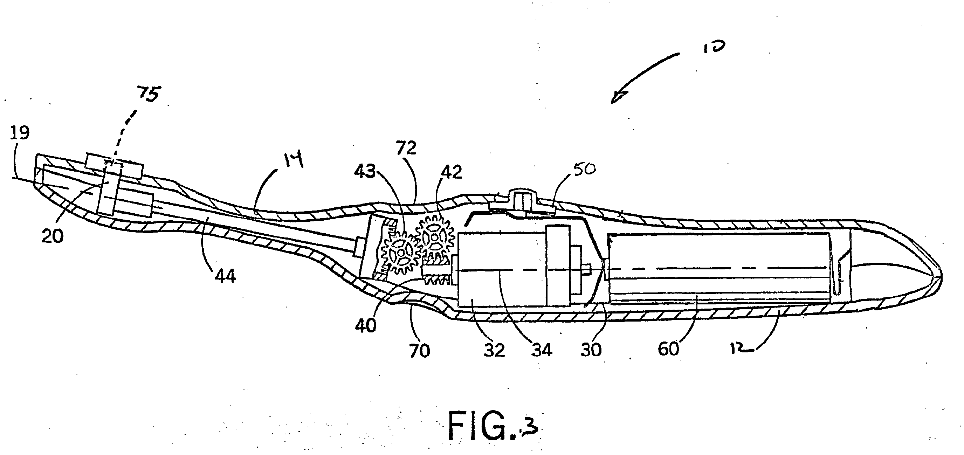

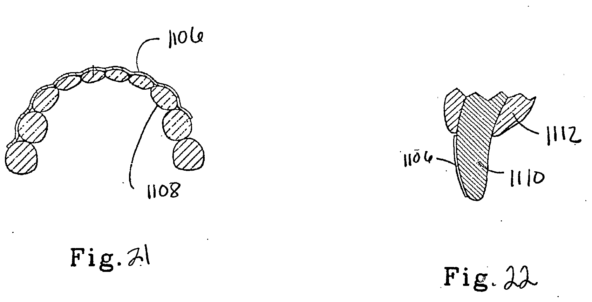

Referring now to **FIG. 2,** the head **16** further is defined by a longitudinal axis **19,** and comprises a moving bristle holder **20** and one or more optional static bristle holders **22**. In this embodiment the static bristle holders **22** are located on opposite sides of the moving bristle holder **20**. The moving bristle holder **20** in this embodiment is located at the center of the head **16**. The moving bristle holder **20** includes a plurality of bristles **24** supported and retained on the holder **20**. The moving bristle holder can oscillate or rotate about an axis of motion approximately normal to the longitudinal axis **19** of the head **16,** although other motions may be provided as previously described. As described in greater detail herein, disposed along this axis of motion of the moving bristle holder, is an electrically powered element **74.** In a particular embodiment (as shown in **FIG. 2a**), the electrically powered element is a light-emitting element **75** such as a light emitting diode positioned on the head of the toothbrush and generally below or under where the surface of the light emitting element does not extend beyond the bristle bearing surface of the moving bristle holder (as shown in **FIG. 22**). This embodiment of the toothbrush also has gripping portions **70** and **72**. As shown in **FIG. 3,** the handle **12** further includes a hollow portion **30** which houses a motor **32,** and has a longitudinal axis **34**. The motor **32** powers the moving bristle holder **20** through a rotatable shaft **44**. A gearing arrangement is operatively interconnected between the shaft **44** and the motor **32**. The gearing arrangement includes a worm gear **40** and a pair of step gears **42, 43**. The motor **32** is operatively connected to the worm gear **40**. Step gear **42** is operatively connected to step gear **43** and the worm gear **40**. A light emitting element **75** is provided that is disposed in the interior of the moving bristle holder **20**. As used herein, the term "light-emitting" element is intended to refer to an element that converts electrical energy into light, as opposed to an element that merely conducts or transmits light, such as a fiber optic cable or wire. In one embodiment the light-emitting element of the present invention is a light emitting diode or LED. For light emitting diodes, the dominant or central wavelength can determined by the equations:

$$\lambda_c = \int_{\lambda\min}^{\lambda\max} I(\lambda) \cdot \lambda \cdot dl \Big/ \int_{\lambda\min}^{\lambda\max} I(\lambda) \cdot d\lambda$$

For continuous spectrums, and

$$\lambda_c = \sum_i I_i \lambda_i \Big/ \sum_i I_i$$

For discrete spectrums.

Wherein I is illumination intensity and $\lambda$ is wavelength.

These equations are further described in CIE 127 (1997) entitled "Measurement of LEDs", which is published by the International Commission of Illumination. These equations and methodology can be also be applied to light-emitting elements other than LEDs, or other methodologies and equations known in the art can be utilized to determine the dominant or central wavelength of a light-emitting element. The spectral (e.g., peak wavelength), photometric (e.g., luminous intensity), radiometric (e.g., radiant intensity), and colormetric (e.g., dominant wavelength) characteristics of the light-emitting elements can be measured using devices known in the art, such as OL 730CV Radiometer/Photometer manufactured by Optronic Laboratories, Inc. of Orlando, FL Some light may not have a dominant or central wavelength (e.g., white light).

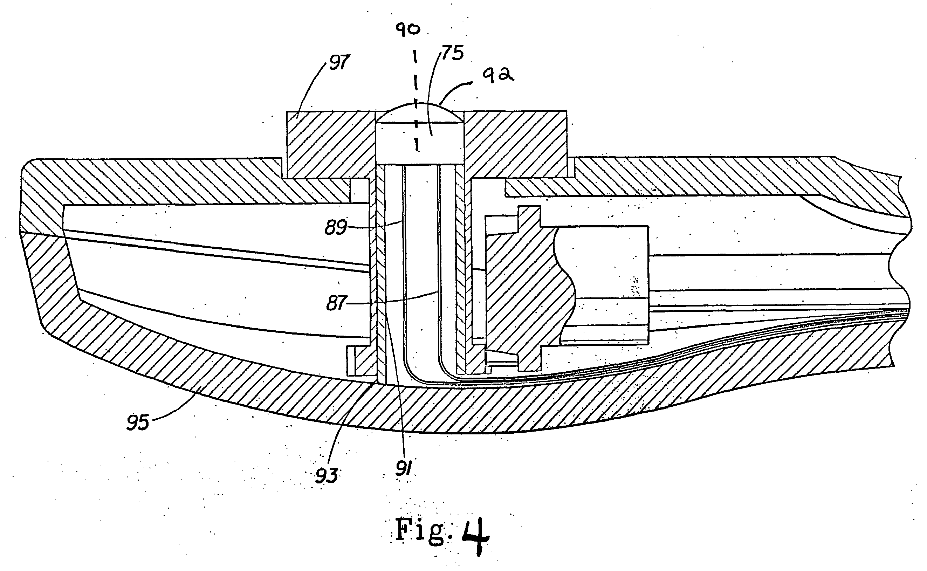

**FIG. 4** illustrates an embodiment of the toothbrush having a stationary light emitting element **75** that is connected to and/or disposed within a pillar **91** that is stationary and fixed to the head **95** at point **93** of the toothbrush. In this embodiment the moving bristle holder **97** oscillates or rotates around the stationary light emitting element **75** disposed within pillar **91**. This light emitting element **75** disposed within the pillar **91** serves as the axis of rotation for the moving bristle holder **97** on the head **95** of the toothbrush. The positive lead **87** and the negative lead **89** can run from the light emitting element **75** through the pillar **91** and then down the length of the head **95** and neck (not shown) of the toothbrush to the power source (not shown).

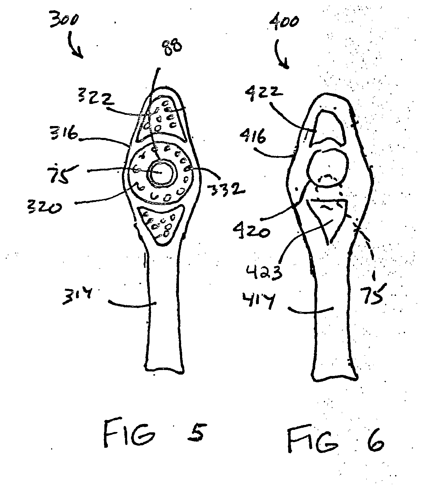

In another embodiment, the light-emitting element **75** is disposed within an aperture or hole **88** that extends through the moving bristle holder **320,** as best seen in **FIG. 5,** so that the light-emitting element is stationary and the moving bristle holder **320** oscillates or rotates about the stationary light-emitting element **75**. In this embodiment, the light-emitting element **75** is fixedly secured to the head **316**. The light-emitting element **75** might extend partially through the hole **88** or it may be disposed below the lower surface of the moving bristle holder **320** so that it is completely contained within the head **316**. The centerline or axis of the light-emitting element **75** may also be the axis of rotation or oscillation for the moving bristle holder **320**. In some of the above-described embodiments, particularly where the light-emitting element is disposed below the movable bristle holder **320,** the moving bristle holder can be formed from a transparent or translucent material. When the light-emitting element is disposed within the head, the light-emitting element may be placed so that it is between bristle holders and not aligned with an axis of rotation/oscillation of a moving bristle holder, as shown by way of example in **FIG. 6,** wherein the bristles have been deleted for clarity. **FIG. 6** illustrates a head **416,** a neck **414,** a movable bristle holder **420,** static bristle holder **422** and **423,** and a light emitting element **75** disposed underneath the movable bristle holder **420** and the static bristle holder **423.** In this embodiment, the top surface of the head and the bristle holders may be formed from a transparent or translucent material.

A variety of materials may be used for forming a transparent or translucent bristle holder and/or head. Examples of such materials include, but are not limited to, polystyrene (PS), polycarbonate (PC), polymethyl methacrylate (PMMA), polyethylene terephthalate glycol (PETG) (commercially available under the designation Eastoman BR003), cellulose acetate propylate (CAP), and combinations thereof. It is contemplated that one or more thermal treatments may be employed to facilitate processing of these materials.

The light-emitting elements can be arranged so that the principle direction of light emission is generally perpendicular to the top surface of the bristle holders and/or generally parallel to the direction of the bristles of the bristle holder. In other words, the light-emitting element can be arranged so that the centerline **90** of the light-emitting element is generally perpendicular to the top surface of the head and/or bristle holder, as best seen in **FIG. 4.** The centerline **90** typically passes through the lens **92** or aperture of the light-emitting element. When the light-emitting element is disposed within, on, or below a moving and/or static bristle holder, a cylindrical region or volume about the centerline **90** of the light-emitting element can be devoid of bristles so that light is transmitted to the brushing surface without interference from the bristles. In one embodiment the diameter of the cylindrical volume that is devoid of bristles is between about 2 mm and about 8 mm, in another embodiment between about 3 mm and about 6 mm. The moving bristle holder still, however, can have at least one ring of bristles that encircle the light-emitting element, as shown by way of example in **Fig. 5.** Additional bristle tufts or an inner ring of bristle tufts might, however, be provided.

Referring again to **FIG. 3,** a switch **50** is provided to control operation of the illuminated electric toothbrush and is operatively connected to the motor **32.** The switch **50** is also configured to operate the one or more lighting elements of the toothbrush. Such operation can be momentary or continuous. When the switch **50** is closed, a circuit is completed between a battery **60** provided within the hollow portion **30** of the handle **10** and the motor **32** and lighting element **75.**

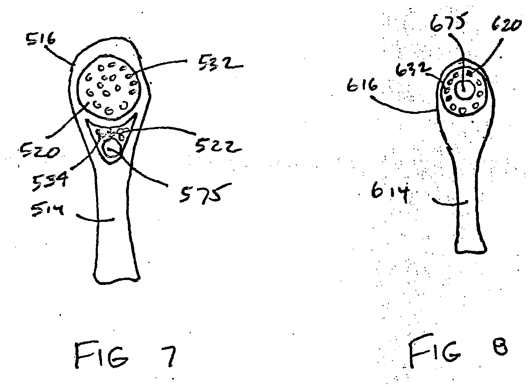

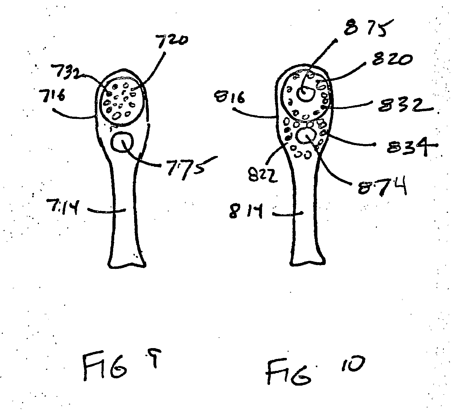

**FIGS. 7-10** illustrate other head, bristle holder and bristle configurations for illuminated electric toothbrushes, all of which contain one or more lighting emitting elements. **FIG. 7** not covered by the present invention illustrates a head **516** and a neck **514.** It will be appreciated that the neck **514** extends between the head **516** and a handle of the toothbrush (not shown). Disposed on the head **516** is a single moving bristle holder **520** having a plurality of bristles tufts **532** disposed thereon. Disposed on a second bristle holder **522** is a light-emitting element **575.** **FIG. 8** depicts another head **616** and neck **614** in accordance with the present invention. The head **616** comprises a single bristle holder **620** comprising bristles **632,** and having a light-emitting element **675** disposed therein. **FIG. 9** not covered by the present invention depicts another head **716** having a single bristle holder **720** disposed thereon and a neck **714.** A light-emitting element **775** is disposed adjacent the bristle holder **720** on the head **716.** The light-emitting element **775,** however, is not disposed on bristle holder. **FIG. 10** depicts still another head **816** having a first bristle holder **820** that moves, a second bristle holder **822** that is fixed or stationary, and a neck **814** connected to the head **816.** Both bristle holders have light-emitting elements **875** disposed thereon. The first bristle holder **820** has a plurality of bristle tufts **832** that encircle the light-emitting element **875** disposed thereon, and the second bristle holder **822** has a plurality of bristle tufts **834** that encircle the light-emitting element **874** disposed thereon.

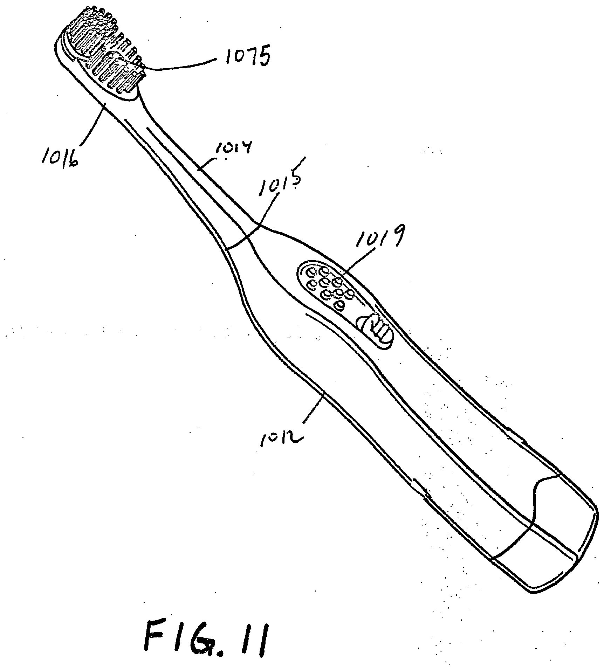

Another embodiment of an electric toothbrush made according to the present invention is shown in **FIG. 11,** having a head **1016,** neck **1014,** and a handle **1012.** Disposed on the head **1016** is a light-emitting element **1075.** The neck and handle are releasably connected at **1015** and contain corresponding structures for their physical engagement and for establishing electrical communication between the lighting-emitting element and the power source. This embodiment of the invention also comprises a gripping portion **1019.**

A wide variety of light-emitting elements may be used with the present invention. In one embodiment the lighting-emitting element is a small, low power consumption, light emitting diode (LED) such as those commercially available under the designation Luxeon™ manufactured by Lumileds Lighting, LLC of San Jose CA. Other commercially available lighting units include those from American Opto Plus LED Corporation. The LED can operate from a relatively low voltage DC power supply, such as in one embodiment between about 0.5 volt and about 5 volts, an in another embodiment between about 1 volt and 3 volts, and in another embodiment between about 1.6 to about 2.4 volts.

The various embodiment toothbrushes described herein may utilize lighting-emitting elements having a variety of characteristics. The electric toothbrushes described herein can emit light having a central wave length between about 10 nm and about $10^6$ nm, and in one embodiment from about 390 nm to about 770 nm, and in another embodiment from about 420 nm to about 490 nm, and for a blue light between about 420 nm and about 470nm.

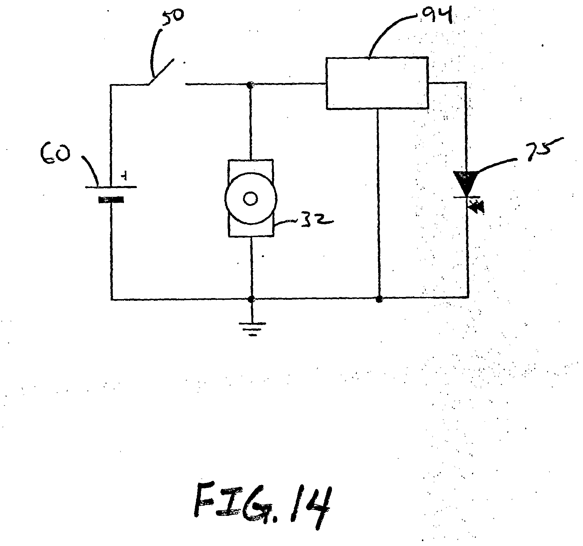

**FIG. 14** illustrates a schematic of an electrical configuration for the present invention. In this configuration, the light-emitting element **75** and the motor **32** are powered or activated concurrently with one another by switch **50.** When the light-emitting element **75** is an LED, it may be desirable to include a voltage or current driver **94** which provides a constant voltage or current output to the LED despite changes to the input voltage or current, especially as the voltage or current output from a battery tends to decrease over time. A voltage or current driver suitable for use with the present invention is the ZXSC310 Single or Multi Cell LED Driver manufactured by Zetex Semiconductors, Oldham, UK. Other embodiments of the invention include, for example, separate switches can be provided to separately active the light-emitting element and the motor. Additionally, more than one light-emitting element might be provided. Light-emitting elements having different spectral, photometric, radiometric, and colormeteric characteristics (e.g., different dominant wavelengths, peak wavelengths, radiometric power, etc.) might be provided to accommodate multiple uses in a single electric toothbrush.

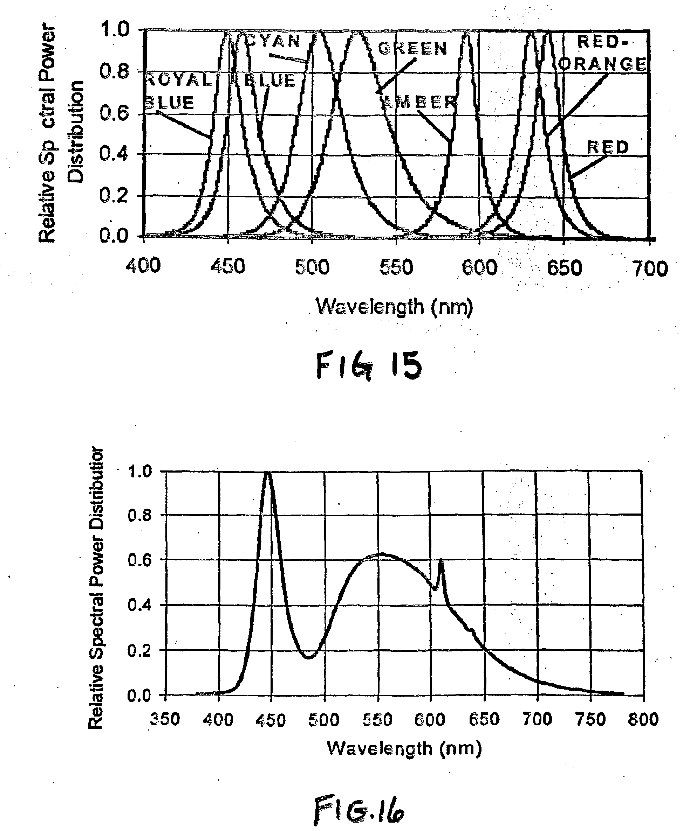

**FIGS. 15** and **16** illustrate spectral distributions for various colors of commercially available LED lighting unit used in the electric toothbrushes described herein. These spectral distribution graphs are for Luxeon™ 1- watt emitter lighting elements, however these distribution patterns may be achieved with other lighting units. Specifically, **FIG. 15** is a graph of the relative spectral power distribution for various colors light emitting elements. **FIG. 15** illustrates the colors of royal blue, blue, cyan, green, amber, red-orange, and red. **FIG. 16** is the relative spectral power distribution for a white color lighting element.

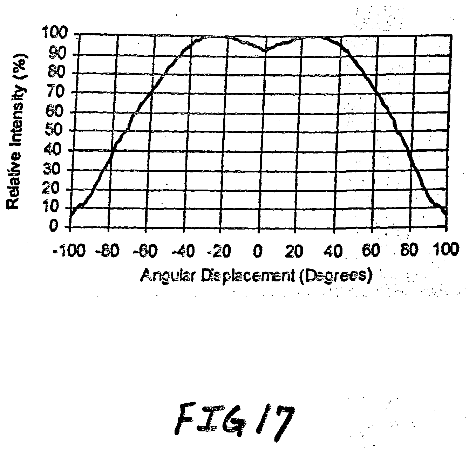

It is often desirable to utilize a light emitting element that provides a generally or substantially uniform distribution of light so that each tooth receives about the same amount of light over the tooth surface. Therefore, lambertian or bell-shaped light patters such as shown by way of example in **FIG. 17** can be used. As discussed above, however, the light emitting element may provide a wide variety of light radiation patterns in accordance with the present invention.

## B. Substances For Use in Combination with the Oral Care Implement

Teeth are composite biological structures. For the purposes of stain removal, the important part of the tooth structure is the crown. The outer layer of the crown consists of enamel, which is a calcified structure that varies from translucent to yellow-gray in color. Underneath the enamel is the dentin, and then a central core chamber of pulp. Both the enamel and dentin layers are porous. Stain may migrate in these pores by diffusion due to the dynamic environment in the oral cavity from the secretions of the salivary glands.

Tooth discoloration that consumers experience in teeth is largely due to color bodies in the tooth structure itself and secondarily due to accumulated extrinsic stains from dietary tannins, which are often trapped in calculas as well. Discoloration of teeth occurs in both the enamel and dentin layers. The apparent color of the enamel-covered crowns is in part, the result of the color of the underlying dentin. Discoloration may also arise from calculus, which is the mineralized bacterial dental plaque on enamel surfaces. Tooth stains are normally due to porpyrin compounds (derivatives of porphin) originating from dietary habit and food components. They may be generated by mouth bacteria and may be accumulated under the enamel. Removal of extrinsic and intrinsic stains is important for achieving a high degree of whitening that is clinically measurable and consumer noticeable.

When the light emitted by the device is directly absorbed by the colored bodies present on and/or inside the tooth structure, the colored bodies ("chromophores") enter an excited state. When in their excited state these chromophores undergo chemical reactions resulting in loss of color and/or ease of their removal. Alternatively, the photoreactive pathways may be initiated by having a photosensitizer which is able to absorb the incident light energy and in its excited state transfer energy to the chromophores of the tooth structure and/or to oxygen. The activated choromophores may react with other chemical reactants or the active oxygen generated may react with the chromophores in their ground state causing them to be less chromogenic. Depending on the conditions employed, the active oxygen species can be singlet oxygen, superoxide, hydroxyl radical, hydroperoxyl radical, endoperoxide or a mixture of the above. The presence of amines or amides, in particular, can enhance the generation of superoxide. Additionally, a range of photosensitizers are known to promote active oxygen chemistry.

Additionally, light can activate stain choromophores (undergo electronic transition), and reduce the activation energy barrier making them more susceptible to peroxide bleaching as well as other cleaning and whitening agents. The activation of chromophores by the light may therefore, enhance the oral care benefit such as tooth bleaching and/or whitening. Similarly, stain chromophores can become more susceptible to abrasive whitening because of light treatment which results in faster and better whitening.

A wide variety of tooth whitening substances may be used in combination with the electric toothbrush described herein, particularly an electric toothbrush comprising a light emitting element. The tooth whitening substances may contain a bleaching agent, an abrasive agent, pH modifiers, chelants, surfactants, enzymes, solvents, polymers and photo-sensitizers or any other agent that acts upon chromophores of the teeth by mechanical or chemical action or a combination thereof. The tooth whitening substance can be provided in the form of a solution, paste, gel, viscous liquid, rinse, solid or other suitable form.

## 1. Bleaching Agents

Bleaching agents include metal ion free peroxides, organic peroxides, and metal ion containing peroxides that generate bleaching actives such as an oxygen radical. Examples of bleaching agents include, but are not limited to, peroxides, metal chlorites, perborates, percarbonates, peroxyacids, persulfates, compounds that form the preceding compounds in situ, and combinations thereof. Examples of peroxide compounds include, but are not limited to, hydrogen peroxide, calcium peroxide, carbamide peroxide, and mixtures thereof. In one embodiment the bleaching agent is carbamide peroxide. Metal chlorites include, but are not limited to, calcium chlorite, barium chlorite, magnesium chlorite, lithium chlorite, sodium chlorite, potassium chlorite, and mixtures thereof. Additional bleaching agents include hypochlorite and chlorine dioxide. In one embodiment the bleaching agent is selected from the group consisting of sodium chlorite, peroxide, sodium percarbonate, oxones, and mixtures thereof. The starting bleach can be aqueous or solid material. Peroxides, for example, penetrate into the pores in enamel and dentin, thereby degrading and removing both intrinsic and extrinsic stains.

The amount of bleaching agent in the whitening or bleaching substance may vary. For example, the bleaching agent could be present in an amount of about 3 to about 60 weight percent, based on the total amount of the tooth whitening substance. If hydrogen peroxide is the bleaching agent, according to one particular embodiment, it may be present in an amount from about 3, 5, 7, 10, 12, 15, 20, 30, 40, 50, 60 and/or less than about 60, 50, 40, 30, 20, 15, 12, 10, 7, 5 weight percent, and in another embodiment from about 7 to about 15 weight percent, based on the total amount of the tooth whitening substance. If carbamide peroxide is the bleaching agent, according to one particular embodiment, it may be present in an amount from about 3, 5, 7, 10, 12, 15, 20, 30, 40, 50, 60 and/or less than about 60, 50, 40, 30, 20, 15, 12, 10, 7, 5 weight percent, based on the total amount of tooth whitening substance. The radiant energy from the light-emitting element can be applied while the substance is in contact with the tooth, however, the light emitting from the light emitting element may also be applied prior to or after application of the tooth whitening substance.

In another embodiment, the whitening substance may be in the form of a multicomponent system. For example, the whitening substance may be sold or supplied as a two-part system. This enables the components to be separated from each other prior to use and may promote increased bleaching efficacy and longer storage times.

In this particular embodiment, the two components, referred to herein as Part 1 and Part 2, can be mixed shortly or immediately before application. It is to be understood that this embodiment is intended to cover formulations comprising more than two components. The whitening substance may still be used more than 30 minutes after mixing, but, due to peroxide decomposition, some or most of its whitening effectiveness may be absent.

The first component, Part 1, can be of a gel or paste consistency. Thickeners and/or fillers may be added to achieve this consistency. Part 1 can comprises one or more metal peroxides, in particular those of monovalent or divalent metals. Examples of peroxides include calcium peroxide, zinc peroxide, and sodium peroxide, with other peroxides including, but not limited to, those of potassium, magnesium, and strontium also being suitable for use. In one embodiment the peroxide is suspended or dispersed in a medium to form a mixture which is from about 5% to about 40% metal peroxide by weight. In another embodiment the peroxide is from about 15 to about 30% peroxide by weight, and in another embodiment the peroxide is about 20%. In an alternative embodiment, the mixture is from about 2% to about 16% peroxide by weight, and in another embodiment the peroxide is from about 6% to about 10% peroxide by weight. The component may further comprise one or more additives to modify rheology, texture, flavor, fragrance, color, or other properties. Examples of additive components for use in Part 1 include glycerin, propylene glycol, polyethylene and/or polypropylene glycols, water, and mixtures of the foregoing. In some embodiments alcohol is added to the media.

In an alternate embodiment, the first component, the metal peroxide of Part 1, is suspended or dispersed in a liquid to form a mixture which can be from about 8% to about 25% by weight of peroxide, and in another embodiment from about 8% to about 15% by weight of peroxide.

Part 2 comprises a solution of one or more acids in water or aqueous solution which may be modified to achieve a desired consistency, such as that of a gel or paste, by the addition of thickeners and/or fillers. Acids suitable for use in the present invention include organic acids including acetic acid, tartaric acid, phosphoric acid, and citric acid. The total acid concentration in Part 2 can be from about 30% to about 100% of the stoichiometric requirement to convert metal peroxides to their salts and hydrogen peroxide, and in another embodiment from about 50% to about 80% of the stoichiometric requirement. Examples of thickening agents include xanthan gum, polyacrylic acid, and cellulose derivatives (e.g. carboxymethylcellulose) and examples of fillers include silica, diatomaceous earth, alumina, and powdered polyethylene or polypropylene or other polymers. The thickeners and/or fillers are added in a quantity sufficient to achieve the desired consistency. These same thickeners and fillers may also be used as additives in Part 1. Additives to modify rheology, texture, flavor, fragrance, and color may also be present in Part 2. In addition, alcohol or other water miscible solvents may be added to Part 2.

Parts 1 and 2 can be mixed in equal proportions to form the whitening formulation, although the ratio may vary from 1:1 depending upon the concentrations of the peroxide and the acid.

Once combined, the peroxide of Part 1 reacts with the aqueous acid of Part 2 to generate hydrogen peroxide *in situ.* The whitening substance utilized in conjunction with the illuminated toothbrushes described herein may also contain other appropriate additives such as stabilizing agents, boosters, alkalinizing agents, solvents, aromatizing agents, sweeteners, thickeners, adhesives and moisteners. By way of example, alkalinizing agents suitable for use include sodium hydroxide or triethanolamine, although the alkalinizing power may be modified by varying the amount of potassium salt, xylitol, sweeteners such as saccharine or derivatives of cyclamic acid, thickeners such as derivatives of starch, xanthan gum, colloidal silicas and similar substances, and moisteners, such as glycerine. Each one of the alkalinizing additives, aromatizing agents, sweeteners and thickeners can be present in the substance of gel in an amount between about 0 and about 6% by weight with respect to the total of the substance, while the moistener may be present in an amount between about 40 and about 80% by weight with respect to the total of the substance. The pH of light-activated substances of the present invention may be between about 4.5 and about 9.5, in another embodiment between about 5 and about 8, in another embodiment between about 5 and about 7 and in another embodiment between about 5 and about 6.

The light-activated compositions herein may comprise a thickening agent. In one embodiment the thickening agent (or viscosity modifier) can also function to increase retention of the composition on the teeth. The viscosity modifier may further function to inhibit settling and separation of components or control settling in a manner that facilitates re-dispersion and may control flow properties of the composition. A viscosity modifier is particularly useful to keep bleach agents or other oral care active agents that are in particulate form, suspended within the compositions of the present invention. The viscosity modifier is present at a level of from about 0.01% to about 20%, in one embodiment from about 0.1% to about 10%, and in another embodiment from about 1% to about 3%, and in yet another embodiment from about 0.4% to about 5%, by weight of the composition. Suitable viscosity modifiers herein include natural and synthetic polymers and gums such as cellulose derivatives (e.g. methylcellulose, carboxymethylcellulose, hydroxyethylcellulose, hydroxypropylcellulose etc), carbomer polymers (e.g. polyacrylic acid copolymer or homopolymer and copolymers of acrylic acid cross linked with a polyalkenyl polyether), karaya gum, guar gum, gelatin, algin, sodium alginate, chitosan, polyethylene oxide, acrylamide polymers, polyvinyl alcohol, polyamines, polyquarternary compounds, ethylene oxide polymers, polyvinylpyrrolidone, cationic polyacrylamide polymers and mixtures thereof. In one embodiment the thickening agent is selected from carbomers, e.g. the class of homopolymers of acrylic acid crosslinked with an alkyl ether of pentaerythritol or an alkyl ether of sucrose. Carbomers are commercially available from B,F. Goodrich as the Carbopol series. In one embodiment the carbopols are Carbopol 934, 940, 941, 956, and mixtures thereof. In another embodiment the viscosity modifier is a hydrophobically modified carbomer. Hydrophobically modified carbomers can increase the retention of compositions herein and/or integral carriers on tooth surfaces and slow the erosion of the compositions once applied on the tooth surfaces. Suitable hydrophobically modified carbomers include acrylate/C10-C30 alkyl acrylate crosspolymer such as Carbopol 1382, Carbopol 1342, Carbopol 1392, and Carbopol ETD 2020, all available from BF Goodrich, and acrylates/C10-C30 alkyl acrylate crosspolymer such as Pemulen TR-1 and Pemulen TR-2 both available from B.F. Goodrich. In one embodiment mixtures of hydrophobically modified carbomers with carbomers can be used. In another embodiment carboxy functional silicones (diacid, monoacid) are used to increase retention of bleaching agents on teeth.

The present invention may be used in conjunction with nearly any tooth whitening substance and/or substance, such as, but not limited to, the substances described in USPNs 6,488,914; 5,851,514; 4,980,152; 3,657,413; 4,983,380; 5,084,268; 5,171,564; 5,376,006; 5,645,428; 5,713,738; RE 34,196; 5,122,365; 6,558,654; 6,555,020; 6,536,628; 6,533,582; 6,521,215; 6,514,543; 6,479,037; 6,447,757; 5,891,453; 6,555,020; and 6,419,905 and Application Nos. WO 03/007680, and USSN 10/154,020. It is not necessary that the substance exhibit an enhanced whitening function upon exposure to light. Benefits may result simply from exposure of the tooth surface to light from the electrical toothbrush prior to application of the whitening substance. Furthermore, additional benefits may stem from greater brushing or cleaning efficacy resulting from illuminating the brushing area.

## 2. Non-Bleach Tooth Whitening and Stain Removal Agents

Additional actives which provide an oral care benefit, such as whitening and/or stain removal, to the teeth include polymers, solvents, chelants, surfactants, and/or enzymes and mixtures thereof. These actives can activate the chromophores, and when used in combination with light emitting from the head of an electric toothbrush, can result in whitening and/or stain removal. Additionally, some of the actives, such as polymers, can serve as oral care carriers to deliver an active to the surfaces of the oral cavity. Examples of polymers include polyvinylpyrrolidone, vinyl pyrrolidone/vinyl acetate copolymer ("PVP-VA"), Carbopol, Polyox resin, and/or silicones and mixtures thereof. The polymers can be added to the tooth whitening and/or stain removing substances in an amount from about 0, 5, 10, 30, 30, 40, 50, 60, 70, 80, 90 and/or less than about 90, 80, 70, 60, 50, 40, 30, 20, 10, 5 weight percent, based upon the total amount of tooth whitening substance. Examples of solvents include but are not limited to: hexamethyldisilozane ("HMDS"); ethyl acetate ("EtAC"); acetone; poly dimethyl siloxane("PDMS"); hexane; and isododecane and mixtures thereof. Solvents can be added to the tooth whitening and/or stain removing substances in an amount from about 0, 5, 10, 30, 30, 40, 50, 60, 70, 80, 90 and/or less than about 90, 80, 70, 60, 50, 40, 30, 20, 10, 5 weight percent, based upon the total amount of tooth whitening substance. Examples of chelants include, but are not limited to: pyrophosphates, including tetrasodium pyrophosphate ("TSPP") and tetrapotassium pyrophosphate ("TKPP"); glycine ("Gl-H"); ethylenediamine tetraacetic acid ("EDTA"); ethane hydroxy diphosphonate ("EHDP"); and/or nitrilotriacetic acid ("NTA") and mixtures thereof. Chelants can be added to the tooth whitening and/or stain removing substances in an amount from about 0, 2, 3, 5, 10, 30, 30 and/or less than about 30, 20, 10, 5 weight percent, based upon the total amount of tooth whitening substance. Examples of surfactants include, but are not limited to: sodium lauryl sulfate ("SLS"); pluronics; polyethyleneoxide; quaternary ammonium; and/or zwitterionics and mixtures thereof. Surfactants can be added to the tooth whitening and/or stain removing substances in an amount from about 0.1, 2, 3, 5, 10, 30, 30, 40, 50 and/or less than about 50, 40, 30, 20, 10, 5 weight percent, based upon the total amount of tooth whitening substance. Examples of enzymes include, but are not limited: to proteases; carbohydrates; laccase; glucox; and/or papain and mixtures thereof. Enzymes can be added to the tooth whitening and/or stain removing substances in an amount from about 0, 1, 2, 3, 4, 5 and/or less than about 5, 4, 3, 2, 1, 0.5 weight percent, based upon the total amount of tooth whitening substance.

### 3. Photosensitizers

Boosters which facilitate or accelerate the action of a bleaching agent can include abrasives, metal catalysts and photosensitizers. These boosters can be added to the tooth whitening and/or stain removing substance in an amount from about 0, 2, 3, 5, 10, 30, 30, 40, 50, 60 and/or less than about 60, 50, 40, 30, 20, 10, 5 weight percent, based upon the total amount of tooth whitening substance. Suitable abrasives include silica, sodium carbonate, calcium phosphate and mixtures thereof. Metal catalysts include Copper, Iron, Manganese and other transition metal ions. A range of photosensitizers are known to produce active oxygen chemistry. These photosensitizers can absorb and can be activated by light in the wavelength of from about 380 to about 700 nm. Photosensitizers or their precursors are selected from the group consisting of: chlorophyll, in particular chlorophyll a & b, and bacterial chlorophyll; rose bengal; methylene blue; Zn phthalocyanine; porphyrin, in particular hematoporphyrin, uroporphyrin, and tetraphenylporphyrins and their complexes of Zn, Al, Si, Sn, phthalocyanines and their complexes with Zn, Al, Si, Sn and Curcumin.; chlorins, in particular bacterialchlorins; riboflavin; bilirubin; curcumin; EDTA; diethylenetriamine pentacetic acid (DEPTA); NTA; EHDP; ethylenediamine tetra(methylenephosphonic acid); and diethylenetriamine penta(methylenephosphonic acid). Photosensitizers can be added to the tooth whitening substance in an amount from about 0.1, 0.5, 1, 2, 3, 5, 7, 10 and/or less than about 10, 7, 5, 3, 2, 1, 0.5, 0.1 weight percent, based upon the total amount of tooth whitening substance. Superoxide may be generated using any of the above sensitizers in combination with an electron donor such as amines and amides -- EDTA, DTPA, diethylene triamine pentaphosphonic acid, triethanolamine, triethylamine, tryptophan, tyrosine or acetanilide. In another embodiment nanometer scale zinc diode and titanium dioxide may be used as photosensitizers.

In some embodiments, it may be desirable that the illuminated toothbrush and whitening substance be "matched." That is, it is desirable that if the whitening substance exhibits enhanced or accelerated whitening function upon exposure to light of a certain wavelength or range of wavelengths, i.e. a band, then the wavelength of light emitted from the lighting unit of the toothbrushes described herein is the same or substantially so as that certain wavelength. For example, if a particular whitening substance is identified for use with the illuminated toothbrushes described herein, and if that substance exhibits enhanced effects upon exposure to light of a peak wavelength of 430 nm to 470 nm, then the toothbrush to be used in conjunction with that substance can emit light having a wavelength within the range of 430 nm to 470 nm.

### 4. Additional Oral Care Actives

Other oral care actives that can be used with the present invention to provide an oral care benefit include, but are not limited to: stannous ion; anti-microbial agents; anti-plaque agents; anti-inflammatory agents; nutrients such as minerals, vitamins, oral nutritional supplements; antioxidants; anti-viral agents; analgesic and anesthetic agents; H-2 antagonists; and additional actives such as insulin, steroids, herbal and other plant derived remedies, antineoplastics, and anti-gingivitis or gum care agents. These oral care actives can be added to the oral care substance in an amount from about 0.01, 1, 5, 10, 20, 30, 40 and/or less than about 40, 30, 20, 10, 5, 1, 0.5 weight percent based on the total amount of the oral care substance.

### 5. Oral Care Carriers and Gelling agents

The oral care substances disclosed herein can comprise an orally acceptable oral care carrier. Additionally, some of the actives disclosed herein can also act as an oral care carrier. In some embodiments an oral care active such as a polymer can be used as a polymer oral care carrier to deliver improved substantivity of the actives, to further adhere the oral care active to the desired surface of the oral cavity and/or to improve delivery of an oral care active to the desired surface of the oral cavity. For some of the actives, the longer the active remains on the oral care surface, the greater the oral benefit that can be delivered. In one embodiment the oral care active is light activated, and therefore, use of a polymer which increases substantivity of the active on the oral surface allows for more exposure of the oral care active to light. Increased exposure time to the light can result an increase in the oral care benefit. An oral care carrier comprises one or more compatible solid or liquid filler diluents or encapsulating substances which are suitable for topical oral administration, and can improve the delivery of oral care actives to the surfaces of the oral cavity. The oral care carrier should be compatible with the actives used in the substances; "compatible" as used herein, means that the components of the substance are capable of being commingled without interaction in a manner which would substantially reduce the substance's stability and/or efficacy. In particular the oral care carrier can include a polymer carrier, such as those described in U.S. Patent Nos. 6,682,722 and 6,589,512 and U.S. Application Nos. 10/424,640 and 10/430,617. Examples of polymers suitable for use in the present invention include but are not limited to: silicone gums and resins, in particular silicone resins having a molecular weight of from about 1000 to about 10,000; dicarboxy functionalized polyorganosiloxanes; water soluble or water dispersible copolymers prepared by copolymerizing one or a mixture of vinyl pyrrolidone monomers (in particular, copolymers of vinyl pyrrolidone with vinyl acetate, vinyl propionate or vinyl butyrate) with one or mixture of C1-C19 alkyl carboxylic acid C2-C12 alkenyl ester monomer; carbopol; Gantrez; and/or polyvinylpyrrolidone.

In one embodiment of the present invention, the polymer carrier comprises as an essential ingredient at least one siloxane polymer functionalized with carboxylic acid groups, for application to polar surfaces such as teeth, ceramics, skin, fabrics, hair, glass and paper. The substances comprise at least about 0.1% of the carboxy functionalized siloxane polymer in a formulation that effectively deposits the polymer to the treated surface. The present polymers comprise a hydrophobic siloxane backbone and pendant anionic moieties containing carboxy groups and have the ability to deposit onto surfaces from aqueous-based formulations such as cleaning and detergent substances and from essentially non-aqueous based formulations. When applied to a suitable surface, the present substance comprising the carboxy functionalized siloxane polymers forms a substantially hydrophobic coating on the treated surface, the coating having prolonged retention thereon.

The carboxy functionalized siloxane polymers useful in the present invention are believed to attach themselves to polar surfaces and to form a coating thereon by electrostatic interaction, i.e., complex formation between the pendant carboxy groups of the polymer with cations or some other positively charged sites on the treated surface. For example, in the case of oral application it is believed the carboxy groups will interact with the calcium ions present in teeth. In the case of fabrics, the interaction may be with calcium ions or cellulose groups; in the case of hair or skin, with the protein residues; in the case of glass or ceramics, with calcium and other metal ions.

The carboxy groups thus serve to anchor the siloxane polymer backbone onto a surface thereby modifying it to be hydrophobic.

The functional group pendant from the polysiloxane main chain comprises two carboxy groups, resulting in improved deposition and retention of the polymer particularly on surfaces such as teeth that contain positively charged calcium ions. The interaction between the carboxy groups and the tooth surface is electrostatic in nature in which the anionic carboxy groups form a complex with the positively charged calcium ions.

Dicarboxy acid functionalized polyorganosiloxanes useful in the present invention have the formula

$$X(R^4R^5SiO)p(R^6ASiO)qY$$

wherein

the X end group represents a triorganosiloxyl end group of formula $R^1R^2R^3SiO$-, or a Z end group wherein Z represents -OH;

the Y end group represents a triorganosilyl end group of formula -$SiR^3R^2R^1$ or a W end group wherein W represents -H;

$R^1$ to $R^6$, which may be identical or different, each represents a linear or branched C1-C8 alkyl or phenyl radical, preferably methyl;

A represents a dicarboxy acid radical of formula

**E-C(O)OM**

-B-CR'〈

**C(O)OM**

wherein

B represents an alkylene residue having from 2 to 30 carbon atoms, preferably from 3 to 8 carbon atoms, optionally substituted by one or more alkyl radicals having from 1 to 30 carbon atoms,

R' represents a hydrogen atom or an alkyl radical having from 1 to 30 carbon atoms,

E is nil or is an alkylene residue having from 1 to 5 carbon atoms, preferably from 1 to 3 carbon atoms, optionally substituted by one or more alkyl radicals having from 1 to 30 carbon atoms; and

M is H, a cation or an alkyl radical having from 1 to 4 carbon atoms optionally substituted with hydroxy or alkoxy groups;

p is an average value ranging from 0 to 1000, preferably from 0 to 500, more preferably from 5 to 200;

q is an average value ranging from 1 to 100 , preferably from 1 to 50; and

the ratio of the number of Z and W end groups to the total number of end groups X and Y ranges from 0/100 to 75/100, preferably from 0/100 to 30/100.

In one embodiment, the p/q ratio is from 1/3 to 99/1 (corresponding to 1-75% of pendant diacid groups relative to the siloxyl units), in another embodiment the p/q ratio is from 1/1 to 10/1. The products where Z is -OH and/or Y is H, are by-products.

The cation salts of the dicarboxy radical can be alkali metal (sodium, potassium, lithium) salts, alkaline earth metal (calcium, barium) salts, non-substituted or substituted ammonium (methyl-, dimethyl-, trimethyl-, or tetramethylammonium, dimethylpiperidinium) salts or can derive from an alkanolamine (monoethanolamine, diethanolamine, triethanolamine).

In addition to the mono- or diester derivatives of the dicarboxy radical (M = alkyl), the present invention includes the amide and diamide derivatives.

The present dicarboxy functionalized siloxane polymers are generally prepared by a hydrosilylation reaction of a polyalkylhydrogensiloxane and an alpha-olefinic anhydride, the precursor of the dicarboxy A groups, with the aid of an effective amount of a hydrosilylation metal catalyst (platinum), as described for example, in US Patent Nos. 3,159,601; 3,159,662; and 3,814,730, followed by hydrolysis of the anhydride groups.

In particular, with respect to bleach delivery from an oral care substance such as dentifrice or mouth rinse, the present polymers having a hydrophobic polysiloxane backbone and pendant moieties containing dicarboxy groups are uniquely suited to facilitate delivery and retention of the bleaching agent on teeth for a period of time sufficient to provide a noticeable whitening benefit, particularly with repeated use of the substances. The present method of using a substantive polymer to deposit and retain the bleaching agent for a prolonged contact time thus represents a novel approach.

In another embodiment the polymer carrier is a vinyl pyrrolidone (VP) / vinyl acetate (VA) copolymer having 60/40 weight ratio of VP/VA and an average molecular weight ranging from about 1000 to about 1,000,000 available from BASF Corp and ISP. Copolymers having a VP/VA ratio ranging from about 30/70 to about 90/10 are also suitable.

The oral care substance of the present invention can be in many forms, including a gel, and in particular including an aqueous gel. A gel is a high viscosity matrix formed from gelling agents. If a gel form is used, a gelling can be used. The gelling agents that can be used in the present invention are safe for oral use, do not readily dissolve in saliva, and do not react with or inactivate the oral care compounds incorporated into them. Generally the gelling agent is a swellable polymer. Suitable gelling agents for use in the present invention include carboxypolymethylene, carboxymethyl cellulose, carboxypropyl cellulose, poloxamers, carrageenan, Veegum, carboxyvinyl polymers, and natural gums such as gum karaya, xanthan gum, Guar gum, gum Arabic, gum tragacanth, and mixtures thereof. The gelling agent can be added to the oral care substance, and in particular to a tooth whitening substance in the form of a gel in an amount from about 0.1, 1, 2, 3, 5, 7, 10, 12, 15 and/or less than about 15, 12, 10, 8, 7, 5, 3, 2, 1, 0.5 weight percent based upon the total amount of the oral care substance.

## 6. Examples

Unless otherwise indicated, all ingredients of the following examples are expressed as a weight percentage of the composition.

### Toothpaste/Dentifrice Examples

Dentifrice compositions not covered by the present invention are shown below. These compositions are made using conventional methods.

### Example Set 1

| Components | 8A | 8B | 8C | 8D | 8E | 8F |
|---|---|---|---|---|---|---|
| Color FD&C Blue#1 | | 0.300 | | | 0.200 | 0.200 |

(continued)

| Components | 8A | 8B | 8C | 8D | 8E | 8F |
|---|---|---|---|---|---|---|
| Carbomer 956 | 2.000 | | | 2.000 | 0.300 | 0.300 |
| Citric Acid | | | 0.180 | | | |
| Flavor | 0.900 | 1.100 | 1.000 | 0.900 | 1.200 | 0.800 |
| Saccharin | 0.300 | 0.400 | 0.450 | 0.400 | 0.300 | 0.350 |
| Glycerin | 10.000 | 30.000 | 30.000 | QS | | |
| Monosodium Phosphate | | 0.500 | | | 0.590 | 0.500 |
| Trisodium Phosphate | | | | | 1.450 | 1.400 |
| Xanthan Gum | | | | | 0.475 | 0.500 |
| Na Hydroxide (50% soln) | 1.100 | | | | | |
| PEG 40 SDIS | | | 1.240 | | | |
| Poloxamer 407, NF | | 15.000 | 15.000 | 5.000 | | |
| Powdered Polyethylene | | 20.000 | 15.430 | | | |
| Silica | | | | 10.000 | 20.000 | 15.000 |
| Sodium Stannate | | | 0.090 | | | |
| Sodium Fluoride | 0.243 | 0.243 | 0.243 | 0.243 | 0.243 | 0.243 |
| Sorbitol (70% soln) | | | | | 50.000 | 40.000 |
| Sodium Alkyl Sulfate (28% soln) | 3.000 | | | | 4.000 | 5.000 |
| Propylsuccinic Acid Polysiloxane Polymer | | 5.000 | | 3.000 | | 1.000 |
| Propylsuccinic Acid/ Propylene Glycol ester Polysiloxane Polymer | 3.000 | | 2.000 | | 4.000 | |
| Urea Peroxide | 10.000 | | | 4.000 | | |
| Hydrogen Peroxide (35% soln) | | 5.000 | 3.000 | | | |
| Triclosan | 0.300 | | | | 0.300 | |
| Cetyl Pyridinium Chloride | | | 0.530 | | | |
| Vitamin E | | | | | | 2.000 |
| Water, Purified USP | QS | QS | QS | | QS | QS |

## Example Set 2

| INGREDIENT | Formula Wt/Wt% | Formula Wt/Wt% | Formula Wt/Wt% | Formula Wt/Wt% | Formula Wt/Wt% | Formula Wt/Wt% |
|---|---|---|---|---|---|---|
| Saccharin Sodium USP (a) | 0.320 | - | 0.50 | 0.70 | 0.50 | 0.50 |
| Trisodium Phosphate | 1.450 | - | - | - | - | - |
| Xanthan Gum NF | 0.475 | 6.00 | - | - | - | - |
| Sodium Fluoride USP | 0.243 | - | - | - | - | - |
| Carbomer 956/Pemulen | 0.300 | 2.00 | - | - | - | - |

(continued)

| INGREDIENT | Formula Wt/Wt% | Formula Wt/Wt% | Formula Wt/Wt% | Formula Wt/Wt% | Formula Wt/Wt% | Formula Wt/Wt% |
|---|---|---|---|---|---|---|
| Monosodium Phosphate | 0.590 | - | - | - | - | - |
| Sorbitol Soln USP (70%) (b) | 62.242 | - | - | - | - | - |
| Silica Abrasive USP | 20.000 | 5.00 | - | - | - | - |
| Purified Water, USP (b) | 8.980 | - | 60.00 | 24.00 | 60.00 | 60.00 |
| Sodium Lauryl Sulfate 28% Soln | 4.000 | - | - | - | - | - |
| Flavor | | - | - | 1.50 | - | - |
| Dye, FD&C Blue No. 1 Soln (c) | 0.200 | - | - | - | - | - |
| Dimethicone (linear PDMS) 10 cst | - | 80.00 | - | | | |
| PDMS (SE 30) | - | 7.00 | - | - | - | - |
| Ethanol | - | - | 32.50 | - | - | - |
| Sodium Tripolyphosphate | - | - | 2.00 | 5.00 | - | 5.00 |
| 60/40 PVP/VA | - | - | 5.00 | 5.00 | 5.00 | 5.00 |
| Propylene Glycol | - | - | - | 53.00 | 30.00 | 25.00 |
| Sodium Benzoate | - | - | - | 0.32 | - | - |
| Bezoic acid | - | - | - | 0.02 | - | - |
| Poloxamer 407 | - | - | - | 10.00 | 4.50 | 4.500 |
| Papain (Enzyme) | - | - | - | - | 0.002 | - |
| Glucose Oxidase | - | - | - | - | - | 0.005 |
| Total | 100.00 | 100.00 | 100.00 | 100.00 | 100.00 | 100.00 |

## Mouth Rinse Examples

Mouth rinse compositions not covered by the present invention are shown below. These compositions are made using conventional methods.

## Example 1

| Ingredient | Weight % |
|---|---|
| Water | 29.000 |
| Propylene Glycol | 53.459 |
| Sodium Benzoate | 0.320 |
| Benzoic Acid | 0.021 |
| Sodium Saccharin | 0.700 |

(continued)

| Ingredient | Weight % |
|---|---|
| Propylsuccinic Acid Functionalized Polysiloxane (AMW = 1700) | 5.000 |
| Poloxamer 407 | 10.000 |
| Flavor | 1.500 |

## Example 2

| Ingredient | Weight % |
|---|---|
| Water | 24.00 |
| Propylene Glycol | 53.46 |
| Sodium Tripolyphosphate | 5.000 |
| Sodium Benzoate | 0.320 |
| Benzoic Acid | 0.020 |
| Sodium Saccharin | 0.700 |
| 60/40 PVP/VA | 5.00 |
| Poloxamer 407 | 10.00 |
| Flavor | 1.500 |

## Example 3

| Ingredient | Weight % |
|---|---|
| Purified Water | 76.638 |
| Glycerin | 23.000 |
| Flavor (Teaberry) | 0.120 |
| Saccharin | 1.018 |
| CPC | 0.074 |
| Poloxamer 407 | 0.050 |
| FD&C Blue #1 | 0.100 |

## Example 4

| Ingredient | Weight % |
|---|---|
| Purified Water | 49.568 |
| 3% H2O2 | 25.000 |
| CPC | 0.053 |
| Poloxamer 407 | 0.050 |
| PVP/VA | 0.200 |

(continued)

| Ingredient | Weight % |
| --- | --- |
| Sucralose | 0.010 |
| Glycerin | 25.000 |
| Menthol | 0.040 |
| Methyl Salicylate | 0.07 |
| FD&C Blue #1 | 0.009 |

## Gel Examples

Gel compositions not covered by the present invention are shown below. These compositions are made using conventional methods.

## Example 1

| Ingredient | Weight % |
| --- | --- |
| Dimethicone (linear PDMS) 10 cst | 80 |
| PDMS (SE30) | 7 |
| Xanthan Gum | 6 |
| Pemulen | 2 |
| Silicone Dioxide Collodial | 5 |

## Example 2

| Ingredient | Weight % |
| --- | --- |
| Ethanol | 32.50 |
| Water | 60.00 |
| Sodium Tripolyphosphate | 2.00 |
| Sodium Saccharin | 0.50 |
| 60/40 PVP/VA | 5.00 |

## Example 3

| Ingredient | 8A | 8B | 8C | 8D | 8E | 8F | 8G |
| --- | --- | --- | --- | --- | --- | --- | --- |
| Flavor | 0.500 | 0.500 | 0.500 | 0.500 | 0.500 | 0.500 | 0.500 |
| Saccharin | 0.100 | 0.100 | 0.100 | 0.100 | 0.100 | 0.100 | 0.100 |
| Propylsuccinic Acid Polysiloxane Polymer (AMW = 1700) | 80.000 | 25.000 | 70.000 | | | 80.000 | |
| Propylsuccinic Acid Polysiloxane Polymer | | | | 75.000 | 66.000 | | 75.000 |

(continued)

| Ingredient | 8A | 8B | 8C | 8D | 8E | 8F | 8G |
|---|---|---|---|---|---|---|---|
| Urea Peroxide | 10.000 | 15.000 | 20.000 | 15.000 | 15.000 | | |
| Triclosan | | | | | | 3.000 | |
| Cetyl Pyridinium Chloride | | | | | | | 1.00 |
| PEG 600 | QS | QS | QS | QS | QS | QS | QS |

## Example 4

| Ingredient | Weight % |
|---|---|
| Glycerin | 70 |
| Carboxypolymethylene | 5 |
| Carbamide Peroxide | 10 |
| Water (pH 6.5) | 15 |

### C. Oral Care Substance Delivery Devices

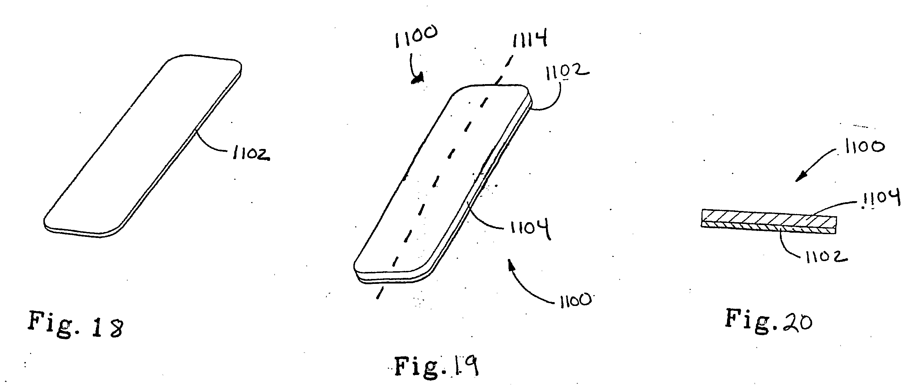

A variety of devices exist for delivering an oral care substance to the surfaces of the oral cavity including, but not limited to, strips of material, trays, and/or paint-on applicators. According to the present invention, as shown in **FIG. 18,** an oral care substance is delivered to an oral surface by a delivery system **1100** comprising a strip of material **1102** which is substantially flat prior to use. Applied or coated onto the strip of material is an oral care substance **1104** as shown in **FIG. 19.** The oral care substance **1104** can be uniform and continuously coated onto the strip of material **1102** as shown in **FIG. 20.** Alternatively, the oral care substance **1104** can be a laminate or separated layers of components, an amorphous mixture of components, separate stripes or spots or other patterns of different components, or a combination of these structures including a continuous coating of oral care substance along a longitudinal axis **1114** of a portion of the strip of material **1102. FIGS. 21** and **22** show a delivery system **1106** applied to a surface of a tooth **1110** and plurality of adjacent teeth **1108.** The tooth **1110** is shown embedded in adjacent soft tissue **1112.** Adjacent soft tissue is herein defined as soft tissue surfaces surrounding the tooth structure including: papilla, marginal gingival, gingival sulculus, inter dental gingival, gingival gum structure on lingual and buccal surfaces up to and including mucogingival junction and the pallet. In both **FIGS. 21** and **22** the delivery system **1106** comprises a strip of material **1102** and further comprises the oral care substance **1104** on the side of the strip of material facing the adjacent plurality of teeth **1108** or single tooth **1110.** The oral care substance may be pre-applied to the tooth and then covered by the strip of material, or the oral care substance can be applied to the strip of material and the applied to the teeth. The delivery system **1100** is used on the oral surface for from 1 minute to about 8 hours. In another embodiment the strip is used on the teeth for from about 1 minute to about 120 minutes. In another embodiment the strip is used on the teeth for from about 30 minutes to about 60 minutes.

Examples of strips which are suitable for use in the inventive method include, but are not limited to, the strips disclosed in U.S. Patent Nos. 6,096,328, 6,136.297, 6,045,811, 5,989,569, 5,894,017, 5,891,453, 5,879,691, 6,277,458, 6,287,120 and 6,343,932.

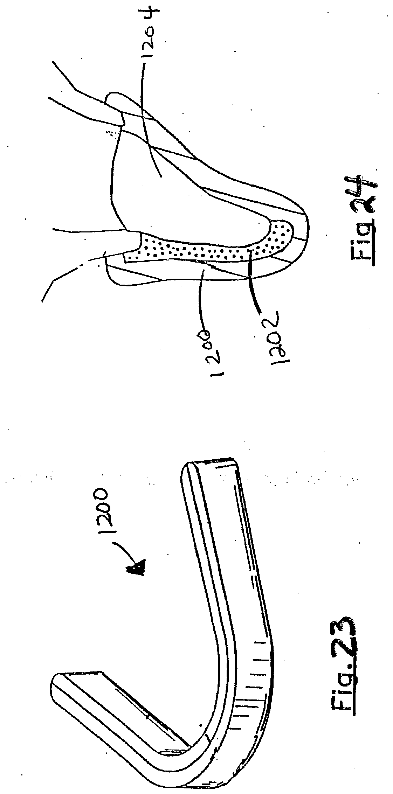

The oral care substance can also be provided to the oral surfaces with a bleaching tray, which, however, is not covered by the present invention such as bleaching trays. A tray **1200** (as shown in **FIG. 22-23**) can be filled with an oral care substance **1202** and placed against the teeth **1204.**

Not covered by the present invention, an applicator can be used to paint-on the oral care substance to the desired surfaces of the oral cavity. The delivery devices can comprise one for the upper teeth and one for the lower teeth. The delivery devices can be disposable, or reusable.

### D. Methods for Delivering Oral care Benefit

The electric toothbrush of the present invention can be used to deliver an oral benefit when used alone or in combination with an oral substance. In some embodiments the teeth are pre-treated with the oral care substance. This pre-treatments allows the oral care substances to absorb further into the oral care surface, such as the teeth, and therefore can increase the resulting oral care benefit when the oral surfaces are exposed to light.

In one embodiment, the invention includes a method wherein a uniform coating of an oral care substance can be applied onto a delivery device and then the oral care substance with the delivery device can be applied to the desired oral surface, such as a plurality of adjacent teeth, the gums, and/or any other surface of the oral cavity. The delivery device is then removed from the oral surface, leaving behind some amount of the oral care substance on the oral surface. The portion of the oral care substance that remains on the teeth after the strip is removed can be from about 0.1, 0.5, 1, 2, 5, 10, 15, 20, 30, 40, 50, 60, 70, 80, 90% to about 100, 90, 80, 70, 60, 50, 40, 30, 20, 15, 10, 5% of the tooth whitening substance. The teeth are then brushed using the earlier described oral care implement; exposing the surfaces of the oral cavity to the emissions from the head of the oral care implement. Additionally, a dentifrice can be used with the oral care implement to clean the surfaces of the oral cavity. The surfaces of the oral cavity can be cleaned with a dentifrice prior to and/or after the application of the oral care substance if desired.

A method not covered by the present invention includes applying a tooth whitening substance to a delivery device to and applying the substance via the delivery device to a plurality of teeth, or alternatively, applying the substance directly to the teeth and then, if desired, placing a delivery device such as a tray and/or a strip of material over the substance. The tooth whitening substance contains can contain from about 5% to about 50% of a tooth whitening active, and the substance is placed into contact with the teeth. The delivery device can remain on the teeth for from about 2, 4, 6, 8, 10, 15, 20, 25, 30, 35, 40, 45, 50, 55, 60 to less than about 60, 55, 50, 45, 40, 35, 30, 25, 20, 15, 10, 5 minutes. The delivery device is then removed, and at least a portion of the tooth whitening substance remains on the teeth. The portion of the tooth whitening substance that remains on the teeth after the strip is removed can be from about 0.1, 0.5, 1, 2, 5, 10, 15, 20, 30, 40, 50, 60, 70, 80, 90% to about 100, 90, 80, 70, 60, 50, 40, 30, 20, 15, 10, 5% of the tooth whitening substance.

In one embodiment the delivery device is a strip of material with a uniform coating of a tooth whitening substance disposed thereon. The strip of material is applied to the teeth and the delivery device can remain on the teeth for from about 2, 4, 6, 8, 10, 15, 20, 25, 30, 35, 40, 45, 50, 55, 60 to less than about 60, 55, 50, 45, 40, 35, 30, 25, 20, 15, 10, 5 minutes. When the strip of material is removed from the teeth, the strip releases from about 0.1 to about 80% of the tooth whitening substance, leaving a plurality of teeth with a coating of tooth whitening substance disposed thereon. The teeth are then brushed with an electric toothbrush comprising a head, a handle, a movable bristle holder, and a light emitting element which is disposed on and emits light from the head of the toothbrush. The teeth can be brushed with the electric toothbrush for from about 30 seconds, 1 minute, 1.5 minutes, 2, minutes, 4, minutes, 5 minutes, 8 minutes and/or less than about 8 minutes, 5 minutes, 4 minutes, 3 minutes, 2 minutes, 1.5 minutes, 1 minute, 30 seconds. The light emitting element can emit light having a wavelength of from about 420 to about 470 nm. This method can be performed from about 1 to about 4 times a day, for about 1 to about 8 weeks. Additionally, this method can be used to replace an every day oral care regimen, and can be used continuously to reduce and prevent staining of the teeth.

In another embodiment a uniform coating of the tooth whitening substance is disposed on the teeth via a delivery device in the form of a strip, and at least a portion of the tooth whitening substance is allowed to remain on the teeth overnight. The teeth
can be brushed with a lighted electric toothbrush made according to the present invention for from about 30 seconds, 1 minute, 1.5 minutes, 2, minutes, 4, minutes, 5 minutes, 8 minutes and/or less than about 8
minutes, 5 minutes, 4 minutes, 3 minutes, 2 minutes,1.5 minutes, 1 minute, 30 seconds.

In an embodiment not covered by the present invention a rinse is used to treat the surfaces of the oral cavity either prior to and/or after the exposure to the emissions from the electric toothbrush. The rinse comprises a whitening active and a polymer which gives substantivity to the tooth whitening active, and/or helps adhere the tooth whitening active to the surfaces of the teeth. The teeth are then brushed using the earlier described oral care implement; exposing the surfaces of the oral cavity to the emissions from the head of the oral care implement.

The aforementioned methods can be repeated from about 1, 2, 3, 4 to about 5, 4, 3, 2, 1 times a day for from about 1 day to about 8 weeks. Additionally, the aforementioned methods can be used indefinitely, for example in place of an every day oral care regimen. In addition to removing stains, plaque and bacteria, if the methods are used in place of an every day oral care regimen, additional staining of teeth, plaque, and/or caries may be prevented from forming.

## E. Kits

The electric toothbrush is packaged as a kit comprising an oral care substance and/or one or more replaceable heads containing a light-emitting element. The heads can thus be replacements or individually assigned to different members of a family. Color distinction is thus often a part of the different heads in a kit. Although the handle is discussed as battery powered, the invention also includes other well known power supplies such as cords for outlet connection or rechargeable batteries and an associated brush holder/charger (not shown). The kit may further include one or more packaged, light-activated oral substances, such as a packaged tooth whitening composition. Additionally, the kit can include other non-light activated oral care substances and toothbrush heads that do not comprise a light emitting element.

**Claims**

1. A kit comprising:

   an electric toothbrush (10) comprising a handle (12, 1012) and a replacable head (16, 95, 316, 516, 616, 716, 816, 1016), the head comprising a movable bristle holder (20, 320), and a light-emitting electrically powered element (75) disposed thereon;
   a light activated tooth whitening substance (1 104, 1202) coated on a delivery device in the form of a strip.

2. The kit according to Claim 1 wherein the light emitting, electrically powered element (75) is a light emitting diode able to provide light having a wavelength of from 420 to 470 nm and a flux density of from 20 to 300 mW/cm$^2$.

3. The kit according to Claim 1 or Claim 2 wherein the tooth whitening substance comprise a photosensitizer activated by light having a wavelength of from 380 to 700 mn, preferably Zn phthalocyanine, and an active selected from hydrogen peroxide, urea peroxide, peroxyacids, peroxyacids precursors, diacyl peroxides, and mixtures thereof.

**Patentansprüche**

1. Kit, umfassend:

   eine elektrische Zahnbürste (10), umfassend einen Griff (12, 1012) und ein austauschbares Kopfstück (16, 95, 316, 516, 616, 716, 816, 1016), wobei das Kopfstück einen beweglichen Bürstenhalter (20, 320), und ein daran angeordnetes lichtemittierendes, elektrisch betriebenes Element (75) umfasst;
   eine lichtaktivierte, zahnaufhellende Substanz (1 104, 1202), die in Form eines Streifens auf einer Abgabevorrichtung aufgebracht ist.

2. Kit nach Anspruch 1, wobei das lichtemittierende, elektrisch betriebene Element (75) eine Leuchtdiode ist, die in der Lage ist, Licht mit einer Wellenlänge von 420 bis 470 nm und einer Flussdichte von 20 bis 300 mW/cm$^2$ bereitzustellen.

3. Kit nach Anspruch 1 oder Anspruch 2, wobei die zahnaufhellende Substanz einen Fotosensibilisator, der durch Licht mit einer Wellenlänge von 380 bis 700 mn aktiviert wird, vorzugsweise Zn-Phthalocyanin, und einen Wirkstoff umfassen, der aus Wasserstoffperoxid, Harnstoffperoxid, Peroxysäuren, Peroxysäuren-Vorläufern, Diacylperoxiden und Mischungen davon ausgewählt ist.

**Revendications**

1. Trousse, comprenant :

   une brosse à dents électrique (10) comprenant un manche (12, 1012) et une tête remplaçable (16, 95, 316, 516, 616, 716, 816, 1016), la tête comprenant un support de poils mobile (20, 320) et un élément luminescent alimenté électriquement (75) disposé sur celui-ci ;
   une substance de blanchissement des dents activée par de la lumière (1 104, 1202) revêtue sur un dispositif d'administration sous la forme d'une bande.

2. Trousse selon la revendication 1, dans laquelle l'élément luminescent alimenté électriquement (75) est une diode électroluminescente apte à fournir une lumière ayant une longueur d'onde allant de 420 à 470 nm et une densité de flux allant de 20 à 300 mW/cm$^2$.

3. Trousse selon la revendication 1 ou la revendication 2, dans laquelle la substance de blanchissement des dents comprennent un photosensibilisant activé par de la lumière ayant une longueur d'onde allant de 380 à 700 mn, de préférence de la phtalocyanine de Zn, et un agent actif choisi parmi le peroxyde d'hydrogène, le peroxyde d'urée, des peroxyacides, des précurseurs de peroxyacide, des peroxydes de diacyle et des mélanges de ceux-ci.

FIG.1

EP 1 663 059 B1

FIG. 2

FIG. 2a

FIG.3

EP 1 663 059 B1

Fig. 4

FIG 5    FIG 6

FIG 7

FIG 8

FIG 9          FIG 10

FIG. 11

FIG. 14

FIG 15

FIG. 16

FIG 17

Fig. 20

Fig. 19

Fig. 18

33

Fig. 22

Fig. 21

EP 1 663 059 B1

1200

**Fig. 23**

1204

1200

1202

**Fig 24**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030059738 A1
- US 2003104340 A1
- US 5989569 A
- US 20030126699 A
- US 20030084525 A
- US 20030084524 A
- US 20030084526 A
- WO 03063723 A
- WO 03063722 A
- US 6360395 B
- US 5617601 A
- US 20030134567 A
- US 20030163881 A
- US 6488914 B
- US 5851514 A
- US 4980152 A
- US 3657413 A
- US 4983380 A
- US 5084268 A
- US 5171564 A
- US 5376006 A
- US 5645428 A
- US 5713738 A
- US RE34196 E
- US 5122365 A
- US 6558654 B
- US 6555020 B
- US 6536628 B
- US 6533582 B
- US 6521215 B
- US 6514543 B
- US 6479037 B
- US 6447757 B
- US 5891453 A
- US 6419905 B
- WO 03007680 A
- US 10154020 B
- US 6682722 B
- US 6589512 B
- US 424640 A
- US 10430617 B
- US 3159601 A
- US 3159662 A
- US 3814730 A
- US 6096328 A
- US 6136297 A
- US 6045811 A
- US 5894017 A
- US 5879691 A
- US 6277458 B
- US 6287120 B
- US 6343932 B

**Non-patent literature cited in the description**

- Measurement of LEDs. CIE. International Commission of Illumination, 1997, vol. 127